# EUROPEAN PATENT SPECIFICATION

(11) **EP 0 936 918 B1**
(45) Date of publication and mention of the grant of the patent: **27.02.2002**
(21) Application number: 97942106.2
(22) Date of filing: 18.09.1997
(51) Int. Cl.: A61K 38/13

(54) **COMBINATION THERAPY FOR TREATMENT OF ARTHRITIC DISEASES**
KOMBINATIONSTHERAPIE FÜR DIE BEHANDLUNG VON ARTHRITIS ERKRANKUNGEN
TRAITEMENT COMBINE DES AFFECTIONS ARTHRITIQUES

(30) Priority: 20.09.1996 GB 9619631
(43) Date of publication of application: 25.08.1999
(73) Proprietor: BRITISH BIOTECH PHARMACEUTICALS LIMITED, Cowley Oxford, OX4 5LY (GB)
(72) Inventor: WOOD, Lars Michael, Cowley, Oxford OX4 5LY (GB); LABER, David Olum, Cowley, Oxford OX4 5LY (GB); WRIGHT, Annette, Cowley, Oxford OX4 5LY (GB)
(74) Representative: Walls, Alan James
(86) International application number: GB9702604
(87) International publication number: WO9811908

(56) References cited:
- WO-A-94/21625
- LOHI, JOUKO ET AL: "Cyclosporin A enhances cytokine and phorbol ester-induced fibroblast collagenase expression" J. INVEST. DERMATOL. ( 1994 ), 102 (6), 938 -44, XP002050444
- DATABASE BIOSIS BIOSCIENCES INFORMATION SERVICE, PHILADELPHIA, PA, US PANAYI G S ET AL: "The use of cyclosporin A in rheumatoid arthritis: Conclusions of an international review." XP002050445 & BRITISH JOURNAL OF RHEUMATOLOGY 33 (10). 1994. 967-969,

## Description

### Field of the Invention.

This invention relates to the use of a matrix metalloproteinase inhibitor and a cyclosporin in combination for the preparation of an agent for treating mammals suffering from arthritic diseases such as rheumatoid arthritis and other tissue-destructive diseases mediated by excessive metalloproteinase activity.

### Background to the invention.

Degradation of extracellular matrix components is an important aspect of normal physiological processes such as embryonic development and tissue repair. The enzymes responsible for this breakdown are the matrix metalloproteinases (MMPs). Normal cells produce low levels of these MMP enzymes however, these levels can increase dramatically in response to a variety of stimuli resulting in extensive tissue breakdown. Cytokines such as epidermal growth factor (EGF), interleukin-1alpha (IL-1α), interleukin-1beta (IL-1β), platelet derived growth factor (PDGF), tumour necrosis factor-alpha (TNF-alpha) and transforming growth factor alpha (TGF-α) are all potent inducers of MMPs such as collagenase and stromelysin.

MMPs are implicated in the tissue destruction associated with inflammatory diseases such as rheumatoid arthritis and osteoarthritis, and inflammatory demyelinating diseases such as multiple sclerosis. In rheumatoid arthritis, there is a pronounced inflammatory response affecting the synovial tissue which is associated with the infusion of large numbers of macrophages and neutrophils. In addition, the synovium possesses elevated levels of matrix metalloproteinase enzymes such as collagenase, gelatinase B and stromelysin, the levels of which appear to reflect the severity of the condition. In stimulated cells such as those of proliferating rheumatoid synovial tissue, collagenase and stromelysin may comprise as much as 2% of the mRNAs produced by the synovial fibroblasts which correlates with high levels of the secreted MMP enzymes (Brickerhoff and Auble, 1990, Ann. N.Y. Acad Sci. 580:355-374).

As the connective tissue destruction by the MMPs is largely irreversible, their inhibition is the target of most therapeutic strategies. Vincenti et al., review the use of inhibitors of metalloproteinases to treat arthritis (Arthritis & Rheumatism. 37(8):1115-1126, 1994).

The matrix metalloproteinases (MMPs) constitute a family of calcium and zinc dependent endoproteinases which are characterised by the presence in the structure of a catalytic zinc atom. It is now known that there exists a range of metalloproteinase enzymes that include: collagenase- 1(MMP-1), 72 kDa-gelatinase (MMP-2), 92 kDa-gelatinase (MMP-9), neutrophil collagenase (MMP-8), collagenase-3 (MMP-13), stromelysin-1 (MMP-3), stromelysin-2 (MMP-10), stromelysin-3 (MMP-11), macrophage metalloelastase (MMP-12), matrilysin (MMP-7) and MT-MMP. The MMPs are collectively capable of hydrolysing all the proteins of the extracellular matrix. Their mechanism is tightly regulated by cytokine regulation of genetic transcription, secretion in latent form that requires activation once produced, and inhibition by specific inhibitors. Many known MMP inhibitors are peptide derivatives, based on naturally occurring amino acids, and are analogues of the cleavage site in the collagen molecule. The paper by Chapman *et al.* (J. Med. Chem. **36**:4293-4301, 1993) reports some general structure/activity findings in a series of N-carboxyalkyl peptides. Other known MMP inhibitors are less peptidic in structure, and may more properly be viewed as pseudopeptides or peptide mimetics. Such compounds usually have a functional group capable of binding to the zinc (II) site in the MMP. Known classes include those in which the zinc binding group is a hydroxamic acid, carboxylic acid, sulphydryl, and oxygenated phosphorus (eg phosphinic acid and phosphonamidate including aminophosphonic acid) groups.

Two known classes of pseudopeptide or peptide mimetic MMP inhibitors (MMPIs) have a hydroxamic acid group and a carboxylic group respectively as their zinc binding groups. With a few exceptions, such known MMPs may be represented by the structural formula (I) 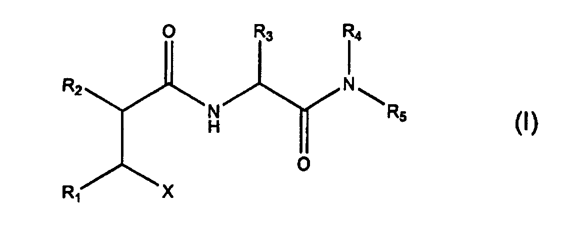 in which X is the zinc binding hydroxamic acid (-CONHOH) or carboxylic acid (-COOH) group and the groups R₁ to R₅ are variable in accordance with the specific prior art disclosures of such compounds. Examples of patent publications disclosing such structures are given below.

In such compounds, it is generally understood in the art that variation of the zinc binding group and the substituents R₁, R₂ and R₃ can have an appreciable effect on the relative inhibition of the metalloproteinase enzymes. The group X is thought to interact with metalloproteinase enzymes by binding to a zinc(II) ion in the active site. Generally the hydroxamic acid group is preferred over the carboxylic acid group in terms of inhibitory activity against the various metalloproteinase enzymes. However, the carboxylic acid group in combination with other substituents can provide selective inhibition of gelatinase (EP-489,577-A). The R₁, R₂ and R₃ groups are believed to occupy respectively the P1, P1' and P2' amino acid side chain binding sites for the natural enzyme substrate. There is evidence that a larger R₁ substituent can enhance activity against stromelysin, and that a (C₁-C₆)alkyl group (such as iso-butyl) at R₂ may be preferred for activity against collagenase whilst an alkylphenyl group (such as phenylpropyl) at R₂ may provide selectivity for gelatinase over the other metalloproteinases.

Tumour necrosis factor-alpha (TNF-α) and transforming growth factor-alpha (TGF-α) are potent pro-inflammatory and immunomodulatory cytokine implicated in inflammatory conditions such as rheumatoid arthritis, Crohn's disease, multiple sclerosis and cachexia associated with cancer or human immunodeficiency virus infection. The ability of these cytokines to induce stromelysin and collagenase expression as mentioned above, may define a pivotal role of these cytokines in the pathogenesis of rheumatod arthritis. The release of mature TNF-α from leukocytes cultured *in vitro* is specifically inhibited by synthetic hydroxamic acid-based metalloproteinase inhibitors, indicating that the processing of the TNF-α precursor is dependent on at least one matrix metalloproteinase-like enzyme, inhibition of which represents a novel therapeutic mechanism for interfering with TNF-α production (Gearing et al. Nature. 370(6490):555-557, 1994). Since this original discovery, a number of cell surface receptors or ligands have been shown to be released in a similar, metalloproteinase inhibitor sensitive manner (growth factors/cytokines)- SCF, M-CSF, EGF, HB-EGF, FasL; (receptors)-TNF RI, TNF RII, IL-6R, IL-1RII, TSHR, CD30, L-selectin, ICAM-1, FLT-3 ligand.

Preventing the production or action of cytokines such as TGF-α, TNF-α, ligands such as FAS or receptors such as IL-6 or CD30 is predicted to be a potent therapeutic strategy for many disease states. These include, but are not restricted to:Crohn's disease, psoriasis, scleroderma, hemangioma, diabetic retinopathy, neovascular glaucoma, retrolental fibroplasia, atherosclerosis, arteriovenous malformations, vascular adhesions, arthritis, wound healing, fibrotic disorders, systemic sclerosis, systemic lupus erythematosus, vasculaities, vasculitides, hypercalcemia of malignancy, ovarian carcinoma, breast cancer, liver cancer, squamous carcinoma, ovarian carcinoma, keratoacanthoma, pancreatic carcinoma, colon carcinoma, erythroleukaemia, viral replication, immune cell disregulation such as occurs in autoimmunity, transplantation rejection and in any disease state where these cytokines are mediators of host injury.

Side effects such as tendonitis have been identified during the clinical trials of some matrix metalloproteinase inhibitors.

The pathological features and clinical symptoms of rheumatoid arthritis (RA) such as acute and chronic inflammation and matrix tissue breakdown are the result of immune responses and metalloproteinase activity. A drug that has proved successful in dampening immune responses and thus the inflammation reaction in rheumatoid arthritis, is cyclosporin A (CyA). Douglas and Torley (Joumal of Rheumatology. 32(Suppl.):57-59, 1993), review the literature conceming the efficacy of CyA in RA.

Cyclosporin(e) is a neutral, lipophilic cyclic endecapeptide (molecular weight 1203) extracted from the fungus *Tolypoclodium inflatum* Gams. For clinical use, cyclosporin (Cyclosporin A; CyA or CsA) is stabilised with castor oil (Cremophor) and olive-oil vehicles for intravenous and oral administration, respectively. CyA is a well established drug in human transplantation which since about 1985, has also been used as an experimental treatment in autoimmune diseases and for the treatment of parasitic infections, in particular protozoal infections such as malaria. Its efficacy in rheumatoid arthritis (RA) and psoriasis/psoriatic arthritis has been demonstrated in numerous placebo-controlled trials (Tugwell et al. Lancet. 335:1051-1055, 1990). CyA is marketed by the Swiss company Sandoz as Sandimmun™ and by the Czech company Galene as Consupren™. Sandoz also market a new oral microemulsion formulation of CyA as Neoral™. CyA binds to intracellular proteins (cyclophilins) which correlates with its immunosuppressive activity. It acts by inhibiting cytokine production involved in T-cell proliferation, particularly of IL-2 and gamma interferon (which provides an amplification signal that activates macrophages and monocytes), resulting in high immunosuppressive activity. CyA therefore inhibits T-lymphocyte-dependent B-cell activation by inhibiting T_{H} and T_{C} cells and reducing gamma-interferon levels. This inhibition of cytokine production most probably reduces the induction of the metalloproteinase enzymes implicated in the matrix tissue degradation associated with diseases such as rheumatoid arthritis and osteoarthritis.

Cyclosporin A has also been successfully used in the treatment of scleroderma, a condition associated with excessive deposition of collagen in the dermis. Lohi et al. (Journal of investigative Dermatology. 102(6):938-944, 1994) demonstrated that cyclosporin A greatly enhances the stimulated expression of collagenase in dermal cells. The authors speculate that CyA may offer a novel approach to the treatment of fibrosis by enhancing degradation of fibrotic connective tissue.

A review of cyclosporin(e), its structure, mechanism of action and its use in drug therapies has been produced, Barry D. Kahan (New England J. of Medicine. Vol 321 No. 25 pp1725-1738; 1989).

Cyclosporin does however have a number of non-immunologic toxic side effects which can hamper its therapeutic use. At therapeutic doses, adverse effects associated with CyA administration include: renal and hepatic dysfunction, gum hypertrophy, hirsutism, fits, hypertension gut-intestinal disturbances, pancreatitis, secondary lymphoma occurrences, hypertension, and muscle weakness.

Thus, whilst cyclosporins, particularly cyclosporin A, are known to be useful in treating inflammatory diseases such as rheumatoid arthritis, their use is hampered by severe dose dependent toxicity problems and consequently, the need for continuous individual patient monitoring. It is a desirable goal therefore, to try to optimise the therapeutic effects of CyA and reduce its toxic effects and possibly the other side-effects. Two potential ways of doing this are (1) to design analogues of CyA which have reduced toxicity yet retained therapeutic efficacy or, (2) to use drug combination therapy. One analogue of CyA from Sandoz, PSC-833, is reported to be approximately 10-fold more potent than CyA (Twentyman PR (1992) Biochemical Pharmacology. 43(1):109-117). Results in animals suggest that the norvaline-cyclosporin analogue (cyclosporin G), in which the alpha-aminobutyric acid at ring position 2 in CyA is substituted by norvaline, possesses less nephrotoxicity than CyA yet similar therapeutic efficacy (Hiestand et al. (1985) Immunology. 55:249-255).

This invention uses a drug combination approach to reduce the toxicity of MMPI and/or CyA administration.

### Summary of the invention

The inventors have discovered, using the art recognised rat adjuvant-induced arthritis model, that co-administration of individually ineffective doses of cyclosporin A and a matrix metalloproteinase inhibitor result in an effective reduction in the clinical indicators of the model. The matrix metalloproteinase inhibitors and cyclosporins therefore, appear to be able to act synergistically in the treatment of inflammatory diseases such as rheumatoid arthritis. This synergy should enable the clinically effective dose of cyclosporin to be reduced. A reduction in the administered dose of cyclosporin will alleviate some of the severe side effects, such as nephrotoxicity, associated with cyclosporin treatment and may also reduce the need for continuous patient monitoring. A reduction in the effective dose of matrix metalloproteinase inhibitors may also alleviate the side effects such as tendonitis associated with their administration.

### Description of the Invention

According to a first aspect of the invention, there is provided the use of a cyclosporin and a matrix metalloproteinase inhibitor in the preparation of an agent for treating mammals suffering from diseases or conditions mediated by excessive metalloproteinase activity, or for use in transplantation therapy.

According to another aspect of the invention, there is provided the use of a cyclosporin and a matrix metalloproteinase inhibitor in the preparation of an agent for treating mammals suffering from arthritic diseases, preferably rheumatoid arthritis, and other tissue-destructive diseases mediated by excessive metalloproteinase activity, said treatment comprising the separate, simultaneous or sequential administration of said matrix metalloproteinase inhibitor and said cyclosporin. According to a preferred aspect of the invention, the MMPI and/or CyA are administered in doses which are respectively ineffective to alleviate arthritis when administered alone but are effective to do so when co-administered.

According to another aspect of the invention, there is provided the use of a cyclosporin and a matrix metalloproteinase inhibitor in the preparation of an agent for treating arthritis by separate, simultaneous or sequential administration of said matrix metalloproteinase inhibitor and said cyclosporin. It is preferred that the MMPI and/or CyA are administered in doses which are respectively ineffective to alleviate arthritis when administered alone but are effective to do so when co-administered.

As used throughout this specification, a matrix metalloproteinase inhibitor (MMPI) is a molecule that is capable of inhibiting at least one of the metalloproteinase enzymes: collagenase-1, stromelysin-1 and 72kDa-gelatinase. An MMPI useful in the invention is one that exhibits biological activity IC₅₀ (50% inhibitory concentration) values of 300nM or less against one or more of collagenase-1, stromelysin-1 and 72kDa-gelatinase.

The potency of compounds as inhibitors of collagenase-1 is determined by the procedure of Cawston and Barrett, (Anal. Biochem. **99**:340-345, 1979), or the following adaptation, whereby a 1mM solution of the compound being tested, or a dilution thereof, is incubated at 37°C for 16 hours with collagen and collagenase-1 (buffered with 25mM Hepes, pH 7.5 containing 5mM CaCl₂, 0.05% Brij 35 and 0.02% NaN₃). The collagen is acetylated ¹⁴C collagen prepared by the method of Cawston and Murphy, (Methods in Enzymology. **80**:711, 1981). The samples are centrifuged to sediment undigested collagen, and an aliquot of the radioactive supematant is removed for assay on a scintillation counter as a measure of hydrolysis. The collagenase-1 activity in the presence of 1mM of the test compound, or a dilution thereof, is compared to activity in a control devoid of inhibitor and the inhibitor concentration effecting 50% inhibition of the collagenase-1 activity (IC₅₀) is obtained. As mentioned above, this assay can be adapted for measuring inhibition of neutrophil collagenase or collagenase-3 by substitution of these enzymes for collagenase-1 above.

The potency of compounds as inhibitors of stromelysin-1 is determined by the procedure of Cawston *et al,* (Biochem. J. **195**:159-165, 1981), or the following adaptation, whereby a 1mM solution of the compound being tested, or a dilution thereof, is incubated at 37°C for 16 hours with stromelysin-1 and ¹⁴C acetylate casein (buffered with 25mM Hepes, pH 7.5 containing 5mM CaCl₂, 0.05% Brij 35 and 0.02% NaN₃). The casein is acetylated ¹⁴C casein prepared by the method of Cawston *et al. (ibid)*. The stromelysin-1 activity in the presence of 1mM of the test compound, or a dilution thereof, is compared to activity in a control devoid of inhibitor and the result of inhibitor concentration effecting 50% inhibition of the stromelysin activity (IC₅₀) is obtained. As mentioned above, this assay can be adapted for measuring inhibition of matrilysin or stromelysin-2, by substitution of these enzymes for stromelysin-1 above.

The potency of compounds as inhibitors of 72kDa-gelatinase is determined by the procedure of Sellers *et al.,* (Biochem. J. **171**:493-496,1979), or the following adaptation, whereby a 1mM solution of the compound being tested, or a dilution thereof, is incubated at 37°C for 16 hours with 50ng ¹⁴C gelatin in 8mM acetic acid, and 72kDa-gelatinase (buffered with 100mM Tris, pH 7.5 containing 5mM CaCl₂, 0.05% Brij 35 and 0.02% NaN₃). The ¹⁴C gelatin is freshly prepared by the thermal denaturation (57°C for 10 minutes) of ¹⁴C-labelled collagen. The collagen is acetylated ¹⁴C collagen prepared by the method of Cawston and Murphy, (Methods in Enzymology. **80:**711, 1981). At the end of incubation, 10µl of 0.5% bovine serum albumin and 80µl of 60% TCA (trichloroacetic acid) are added and the samples placed on ice for 15 minutes. The samples are centrifuged to sediment undigested substrate, and an aliquot of the radioactive supernatant is removed for assay on a scintillation counter as a measure of hydrolysis The 72kDa-gelatinase activity in the presence of 1mM of the test compound, or a dilution thereof, is compared to activity in a control devoid of inhibitor and the inhibitor concentration effecting 50% inhibition of the 72kDa-gelatinase activity (IC₅₀) is obtained.

Schwartz and Van Wart review the state of synthetic inhibitors of bacterial and mammalian interstitial collagenase (Progress in Medicinal Chemistry-Vol. 29 pp 271-334, eds Ellis and Luscombe; 1992) and, JR Morphy et al. (Curr. Medicinal Chemistry. 2:743-762; 1995) reviews the status of matrix metalloproteinase inhibitors as of 1995, and the reader is referred thereto for details of the structures of the compounds disclosed.

As mentioned above, certain pseudopeptide MMP inhibitors have been proposed with hydroxamic acid or carboxylic acid zinc binding groups. The following patent publications disclose such MMP inhibitors:
- US 4599361: (Searie)
- EP-A-2321081: (ICI)
- EP-A-0236872: (Roche)
- EP-A-0274453: (Bellon)
- WO 90/05716: (British Bio-technology)
- WO 90/05719: (British Bio-technology)
- WO 91/02716: (British Bio-technology)
- WO 92/09563: (Glycomed)
- US 5183900: (Glycomed)
- US 5270326: (Glycomed)
- WO 92/17460: (SmithKline Beecham)
- EP-A-0489577: (Celltech)
- EP-A-0489579: (Celltech)
- EP-A-0497192: (Roche)
- US 5256657: (Sterling Winthrop)
- WO 92/13831: (British Bio-technology)
- WO 92/22523: (Research Corporation Technologies)
- WO 93/09090: (Yamanouchi)
- WO 93/09097: (Sankyo)
- WO 93/20047: (British Bio-technology)
- WO 93/24449: (Celltech)
- WO 93/24475: (Celltech)
- EP-A-0574758: (Roche)
- EP-A-0575844: (Roche)
- WO 94/02446: (British Bio-technology)
- WO 94/02447: (British Bio-technology)
- WO 94/21612: (Otsuka)
- WO 94/21625: (British Biotech)
- WO 94/24140: (British Biotech)
- WO 94/25434: (Celltech)
- WO 94/25435: (Celltech)
- WO 95/04033: (Celltech)
- WO 95/04735: (Syntex)
- WO 95/04715: (Kanebo)
- WO 95/06031: (Immunex)
- WO 95/09841: (British Biotech)
- WO 95/12603: (Syntex)
- WO 95/19956: (British Biotech)
- WO 95/19957: (British Biotech)
- WO 95/19961: (British Biotech)
- WO 95/19965: (Glycomed)
- WO 95/22966: (Sanofi Winthrop)
- WO 95/09841: (SmithKline Beecham)
- EP-A-0664284: (Roche)
- WO 95/23790: (SmithKline Beecham)
- EP-A-0684240: (Roche)
- WO 95/32944: (British Biotech)
- WO 95/33709: (Roche)
- WO 95/33731: (Roche)
- WO 95/35275: (British Biotech)
- WO 95/35276: (British Biotech)
- WO 96/06074: (British Biotech)
- WO 96/15096: (Bayer)
- WO 96/16027: (Syntex)
- WO 96/16931: (British Biotech)
- WO 96/17838: (Boehringer)
- WO 96/20918: (Procter & Gamble)
- WO 96/23791: (Syntex)
- WO 96/27583: (Pfizer)
- WO 96/29313: (Procter & Gamble)
- WO 96/33165: (British Biotech)
- WO 96/33166: (DuPont Merck)
- WO 96/33176: (DuPont Merck)
- WO 96/33172: (Pfizer)
- WO 96/33968: (Fuji Yakuhin KKK)
- WO 96/33991: (Sankyo)
- WO 96/35687: (Chiroscience)
- WO 96/35711: (Chiroscience)
- WO 96/35712: (Chiroscience)
- WO 96/35714: (Chiroscience)
- WO 96/38434: (Wamer Lambert)
- WO 96/40101: (Ciba-Geigy)
- WO 97/03966: (British Biotech)
- PCT/GB96/02877: (British Biotech)

The reader is referred thereto for details of the structures of the compounds disclosed and methods for their preparation.

The preferred matrix metalloproteinase inhibitor for use in any aspect of the invention is selected from the group consisting of: 3R-(2,2-Dimethyl-1S-methylcarbamoylpropylcarbamoyl)-5-methyl-2S-2-propenyl-hexanohydroxamic acid (disclosed in Example 5 of WO-A-9421625), [4-(N-Hydroxyamino)-2R-isobutyl-3S-(thiophenylthiomethyl) succinyl]-L-phenylalanine-N-methylamide (disclosed in Example 2 of WO-A-90/05719), and N²-[3S-Hydroxy-4-(N-hydroxyamino)-2R-isobutylsuccinyl]-L-tert-leucine-N¹-methylamide (disclosed in Example 10 of WO-A-94/02447).

Salts of the compounds of the invention include physiologically acceptable acid addition salts for example hydrochlorides, hydrobromides, sulphates, methane sulphonates, p-toluenesulphonates, phosphates, acetates, citrates, succinates, lactates, tartrates, fumarates and maleates. Salts may also be formed with bases, for example sodium, potassium, magnesium, and calcium salts.

As used throughout this specification, cyclosporin refers to the structurally distinctive class of cyclic, poly-N-methylated undecapeptides, commonly possessing immunosuppressive and/or anti-inflammatory pharmacological activity and molecules which have similar actions despite possessing quite different molecular structures, eg. molecules such as rapamycin and FK506 (for a review consult Schreiber and Crabtree. Immunology Today. 13(4)136-142, 1992). Rapamycin and FK506, like CyA, act by binding to endogenous receptors (immunophilins), with the resulting complex targeting the protein phosphatase, calcineurin, to exert the immunosuppressive effect.

The preferred cyclosporin for use in any aspect of the invention is cyclosporin A (CsA or CyA) the first cyclic peptide cyclosporin structure isolated from the fungus *Tolypocladium inflatum* GAMS. The production of cyclosporin A is described in U.S. Pat. No. 4, 117, 118. Other preferred cyclosporin molecules for use in any aspect of the invention are selected from the group consisting of: cyclosporin G, rapamycin and FK506.

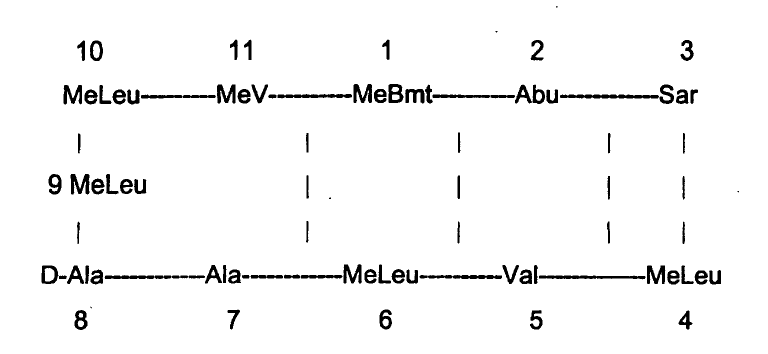

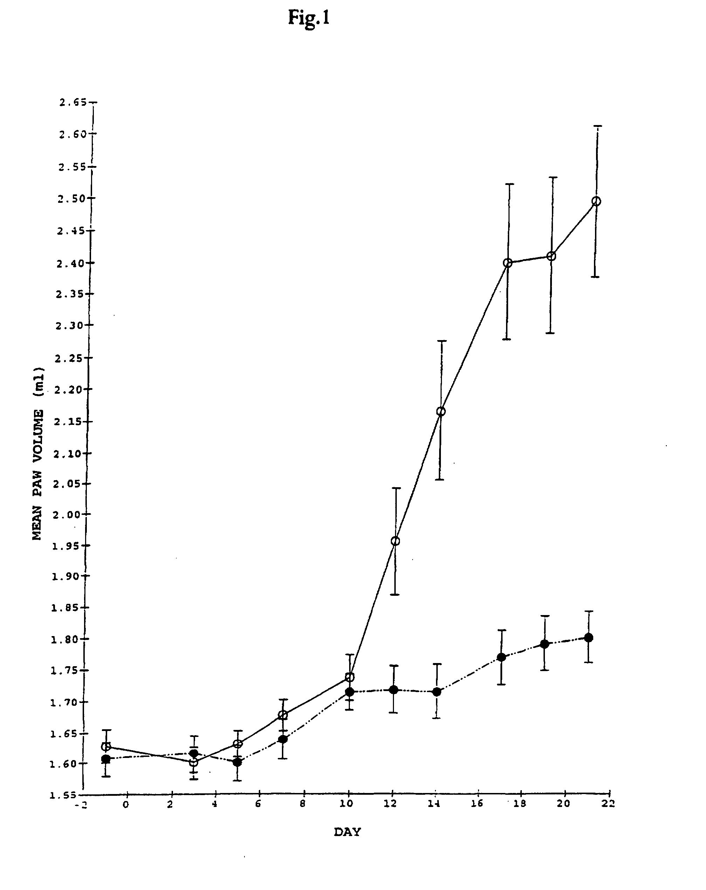

Amino acid alignment of cyclosporin A. The unique structure at position 1 is MeBmt, a novel β-hydroxy, unsaturated, 9-carbon amino acid ((4R)-4-[(E)-2-butenyl]-4, N-dimethyl-L-threonine). Abu denotes α-amino-butyric acid(L-2-amino-butanoic acid); Sar, sarcosine, MeLeu, N-methyl-L-leucine; Val, valine; Ala, L-alanine; D-Ala, alanine; and MeVal, N-methyl-L-valine (taken from Fig. 1 Barry D. Kahan *ibid*).

A wide variety of naturally occurring cyclosporins, from A to Z (von Wartburg et al. Progress in Allergy. 38:28-45, 1986; Kobel et al. Europ. J. Appl. Microbiol. and Biotechnology. 14:273-240, 1981; and Traber et al. 2, Helv Chim. Acta. 65:1655-1667, 1982) have now been isolated and many non-natural cylosporins (derivatives of CyA) have been made usually by total- or semi-synthetic means. A cyclosporin A derivative is one possessing one or more amino acid changes within the 11 amino acid cyclic peptide of CyA. See for example: U.S. Patent Nos: 4, 108, 985; 4, 220, 641; 4, 554, 351; 4, 764, 503; 4, 798, 823 and 5, 284, 826; European patent Nos: 0 056 782, 0 194 972.

A typical example of a cyclosporin A derivative is cyclosporin G (CsG; Sandoz compound OG 37-325) which is reported to have potent immunosuppressive activity but, less nephrotic potential (Tejani et al. 1988, Transplantation. 45:184-187) . CsG is an undecapeptide differing only from CsA in the presence of L-norvaline in place of α-amino-butyric acid (L-2-amino-butanoic acid) at amino acid position-2. The metabolism of CsG is discussed in Mangold et al. Drug Metabolism and disposition. 23(6):615-621.

According to a preferred aspect of the invention there is provided, a pharmaceutical product comprising a matrix metalloproteinase inhibitor and a cyclosporin for separate, simultaneous or sequential use in treating arthritis. It is preferred that the individual doses of said matrix metalloproteinase inhibitor and said cyclosporin present in the product are respectively ineffective to alleviate arthritis when administered alone for treatment of arthritis, but are effective to do so when co-administered.

Disease states or conditions treatable by MMPI and CyA include those such as insulindependent diabetes or multiple sclerosis, Guillan-Barré syndrome, myasthenia gravis, rheumatoid arthritis, periodontitis, scleroderma, Paget's disease, osteoporosis, hyperparathyroidism, cholesteotoma, psoriasis and psoriatic arthritis, cancer, transplantation, comeal ulceration, systemic lupus erythematosus, uveitis, chronic active hepatitis, inflammatory bowel disease or type 1 diabetes. Cyclosporin A is currently routinely used *inter alia,* in the following principal indications: organ transplantation, bone marrow transplantation, psoriasis, atopic dermatitis and rheumatoid arthritis. Tissue-destructive diseases treatable by the invention include: rheumatoid arthritis, osteoarthritis, multiple sclerosis, comeal ulceration, metastatic cancer, periodontal diseases and metabolic bone diseases. The effective dose, administration regime and administration route for treating such diseases is known to those skilled in the art and is well documented.

Cyclosporin blood levels can be measured by immunoassay (e.g. Esa et al., Transplant proc. **20**(suppl 2):80-86., 1988) or high-performance liquid chromatography (HPLC). Renal toxicity is related to blood trough concentration, and it is therefore desirable to keep the trough concentration at or below 100ng/ml, and preferably below 50ng/ml (as measured by HPLC). The co-administration of an MMPI and cyclosporin may allow a therapeutic effect with a lower risk of toxicity by dose reduction and therefore, by keeping the trough concentration below 100ng/ml.

When administered together, the synergistic action of the cyclosporin and the MMPI enables the therapeutic dose of the cyclosporin and/or the MMPI to be lower than that of their individual effective therapeutic doses. Whilst the effective dose of any drug varies from patient to patient, the art established effective dose of cyclosporin A in humans is approximately 2.5mg/kg/day. It is this art established/recommended "effective dose" from which the "ineffective" dose of the agent is gauged. It is a routine task for a person skilled in the art to determine the individual effective dose of any drug, including matrix metalloproteinase inhibitors, undergoing clinical trials in man.

As a guideline for providing the proper amount of agents for treating arthritic diseases such as rheumatoid arthritis and osteoarthritis for implementing the present invention, a rule of thumb is to administer between 1% and 90%, preferably between 20% and 80% of the effective dose of the MMPI and/or the cyclosporin. For the purpose of this specification, the effective individual dose is the recommended dose used for treating the disease state as established following clinical trials.

For current rheumatoid arthritis treatment, the recommended dose of CyA is 2.5mg/kg/day given orally in two divided doses. If the clinical effect is insufficient, the daily dose is increased gradually as tolerability permits, but should not exceed 4-5mg/kg/day orally (refer to Panayi and Tugwell, "The use of cyclosporin A in rheumatoid arthritis" British Joumal of Rheumatology. 33:967-969; 1994). Thus as a guideline for implementing the present invention in the treatment of rheumatoid arthritis, by co-administration of an effective or slightly sub-effective dose of an MMPI, the clinically effective dose of CyA, given to a patient wherein the individual effective dose of CyA is 2.5mg/kg/day, could be between 0.025mg/kg/day to 2.25mg/kg/day, preferably between 0.5mg/kg/day and 2mg/kg/day.

The dosage unit of MMPIs involved in oral administration may contain from about 1 to 250mg, preferably from about 25 to 250mg of a compound of general formula I *supra.* A suitable daily dose for a mammal may vary widely depending on the condition of the patient. However, a dose of a compound of general formula I of about 0.1 to 300mg/kg body weight, particularly from about 1 to 100mg/kg body weight may be appropriate.

Suitable cyclosporins and MMP inhibitors may be prepared for administration by any route. The compositions may be in the form of tablets, capsules, powders, granules, lozenges, liquid or gel preparations, such as oral, topical, or sterile parenteral solutions or suspensions. Tablets and capsules for oral administration may be in unit dose presentation form, and may contain conventional excipients such as binding agents, for example syrup, acacia, gelatin, sorbitol, tragacanth, or polyvinyl-pyrollidone; fillers for example lactose, sugar, maize-starch, calcium phosphate, sorbitol or glycine; tabletting lubricant, for example magnesium stearate, talc, polyethylene glycol or silica; disintegrants for example potato starch, or acceptable wetting agents such as sodium lauryl sulphate. The tablets may be coated according to methods well known in normal pharmaceutical practise. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives such as suspending agents, for example sorbitol, syrup, methyl cellulose, glucose syrup, gelatin hydrogenated edible fats; emulsifying agents, for example lecithin, sorbitan monooleate, or acacia; non-aqueous vehicles (which may include edible oils), for example almond oil, fractionated coconut oil, oily esters such as glycerine, propylene glycol, or ethyl alcohol; preservatives, for example methyl or propyl p-hydroxybenzoate or sorbic acid, and if desired conventional flavouring or colouring agents. Sandimmun™ CyA for intravenous infusion contains polyethoxylated caster oil and is normally diluted 1:20 to 1:100 with normal saline or 5% glucose before use. Intravenous dosages of CyA are usually one-third of oral dosages.

The pharmaceutical product of the invention comprises both the cyclosporin and the matrix metalloproteinase inhibitor compounds in unit doses which are respectively ineffective to alleviate arthritis when administered alone but, are effective to do so when co-administered.. Provided there are no pharmacokinetic interactions, it may be convenient to mix the two components together in appropriate unit doses. However, for stability purposes it may be desirable to keep both components as separate entities for admixture prior to administration or for administration simultaneously, sequentially or separately. It is envisioned that individual tablet or capsule forms of the product can be formulated with the two compounds kept apart by an inert barrier. Such an inert barrier may for example comprise a polymeric membrane that is readily digestible in the stomach. Alternatively, both agents can be prepared in granule or pellet form, mixed together and formulated at the correct doses in the same gelatine capsule. Yet another alternative would be to prepare two-layered tablets comprising the individual unit doses of the components. Various means of formulating the two components separately are available and within the abilities of the person skilled in formulation technology.

The active ingredient may be administered parenterally (eg. subcutaneous, intramuscular and intravenous) in a sterile medium, which is preferably isotonic with the blood of the recipient. Depending on the vehicle and concentration used, the drug can either be suspended or dissolved in the vehicle. Advantageously, adjuvants such as a local anaesthetic, preservative and buffering agents can be dissolved in the vehicle. Among the acceptable vehicles and solvents that may be employed are water, Ringer's solution, and isotonic sodium chloride solution.

For topical application to the skin, the drug(s) may be made up into a cream, lotion or ointment. Cream or ointment formulations which may be used for the drug(s) are conventional formulations well known in the art, for example as described in standard textbooks of pharmaceutics such as the British Pharmacopoeia. Topical preparations of 2% cyclosporin A in Labrafil produce favourable results in chronic dermatitis.

For topical application to the eye, the drug may be made up into a solution or suspension in a suitable sterile aqueous or non aqueous vehicle. Additives, for instance buffers such as sodium metabisulphite or disodium edeate; preservatives including bactericidal and fungicidal agents such as phenyl mercuric acetate or nitrate, benzalkonium chloride or chlorhexidine, and thickening agents such as hypromellose may also be included.

The following examples and figures illustrate the invention, but are not intended to limit the scope in any way.

Figure 1 illustrates the effect of 5mg/kg Cyclosporin A po, bid, treatment, starting on day 10, on the mean paw volume in the rat arthritis model. Open circles represent PBS/Tween 5ml/kg, po, bid. Solid circles represent Cyclosporin A 5mg/kg, po, bid.

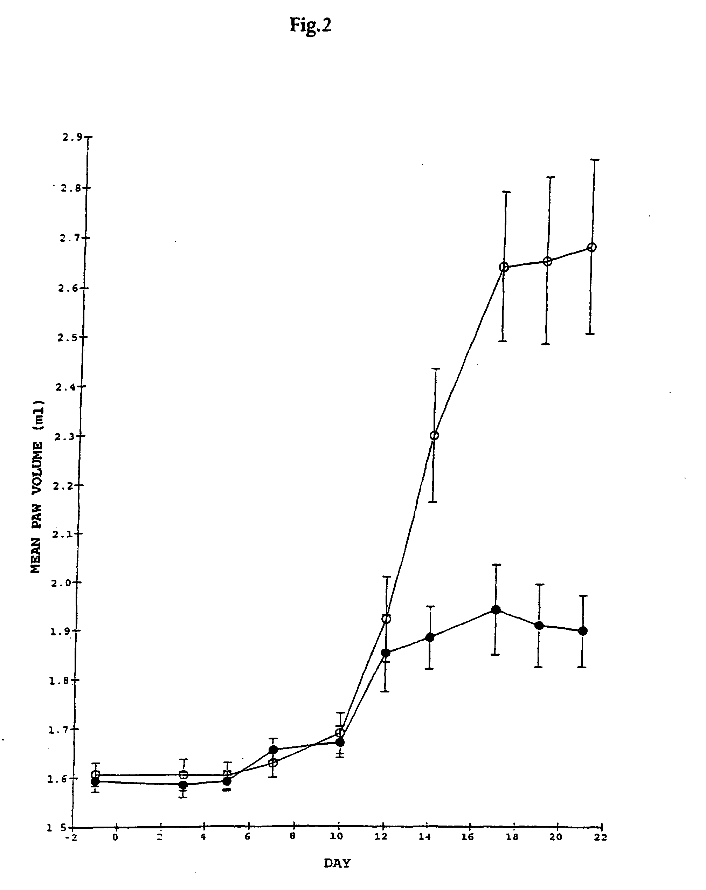

Figure 2 illustrates the effect of 1 mg/kg Cyclosporin A po, bid, treatment, starting on day 10, on the mean paw volume in the rat arthritis model. Open circles represent PBS/Tween 5ml/kg, po, bid. Solid circles represent Cyclosporin A 1mg/kg, po, bid.

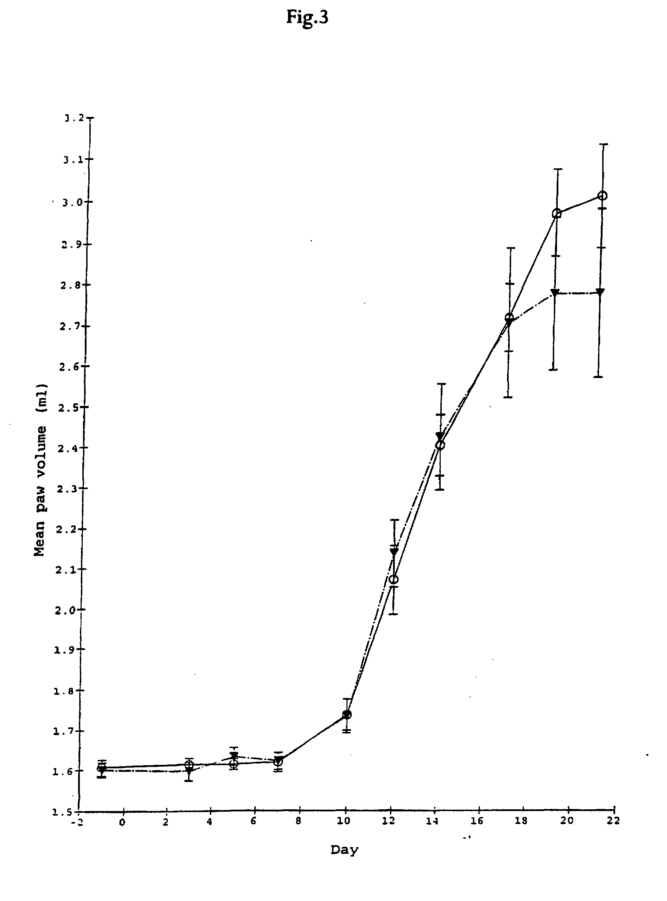

Figure 3 illustrates the effect of 0.3mg/kg Cyclosporin A po, bid, treatment, starting on day 10, on the mean paw volume in the rat arthritis model. Open circles represent PBS/Tween 5ml/kg, po, bid. Solid triangles represent Cyclosporin A 0.3mg/kg. po, bid.

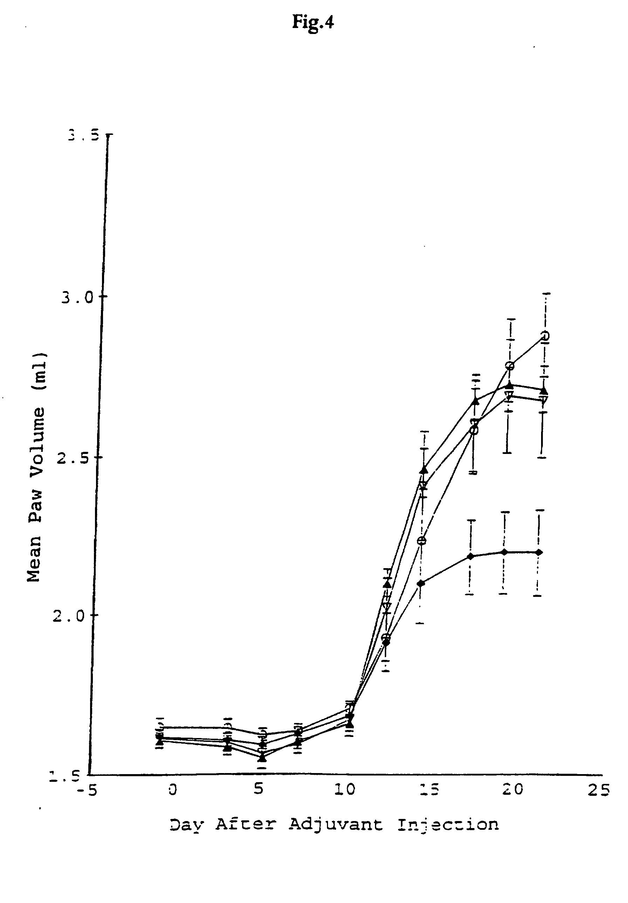

Figure 4 illustrates the effect of combined 0.3mg/kg CyA, po, bid, and 10mg/kg Compound 1, po, bid, treatment, starting on day 10, on the mean paw volume in the rat arthritis model. Open circles represent PBS/Tween 5ml/kg, po, bid. Open triangles represent Cyclosporin A 0.3mg/kg, po, bid. Solid triangles represent compound 1 10mg/kg, po, bid. Solid diamonds represent combined compound 1 at 10mg/kg and Cyclosporin A 0.3mg/kg, administered po, bid.

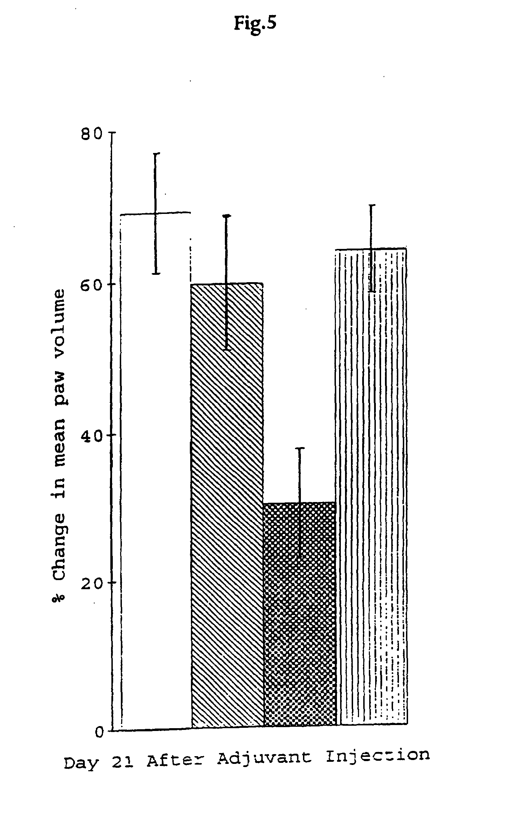

Figure 5 illustrates the effect of treatment, between days 10 and 20, on the change in mean paw volume measured at day 21 in the rat arthritis model. Histogram bars (1-4) from left to right represent: 1) (Open bar) PBS/Tween 5ml/kg, po, bid; 2) (diagonal line bar) Cyclosporin A 0.3mg/kg, po, bid; 3) (check bar) combined compound 1 at 10mg/kg and Cyclosporin A 0.3mg/kg, administered po, bid; and 4) (vertical line bar) compound 1 10mg/kg, po, bid.

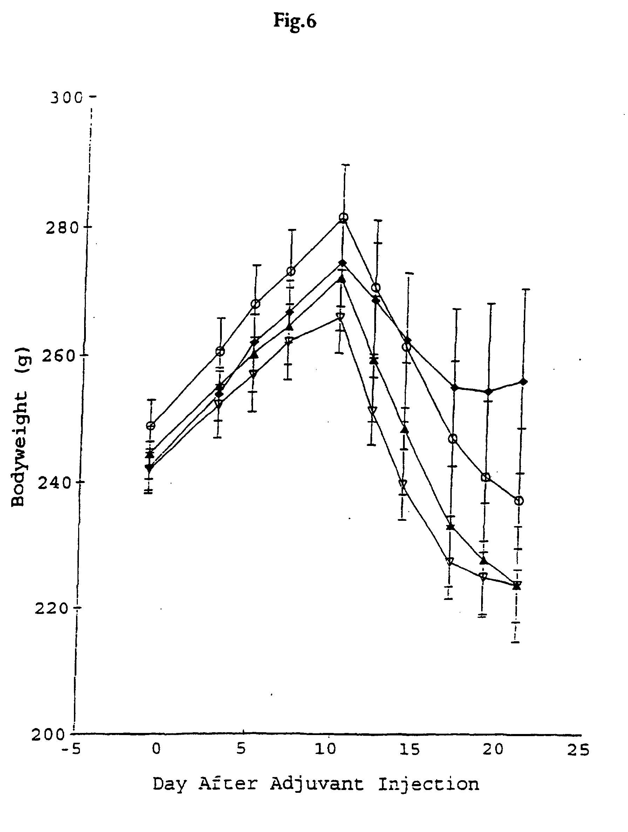

Figure 6 illustrates the effect of treatment, starting on day 10, on the mean bodyweight in the rat arthritis model. Open circles represent PBS/Tween 5ml/kg, po, bid. Open triangles represent Cyclosporin A 0.3mg/kg, po, bid. Solid triangles represent compound 1 10mg/kg, po, bid. Solid diamonds represent combined compound 1 at 10mg/kg and Cyclosporin A 0.3mg/kg, administered po, bid.

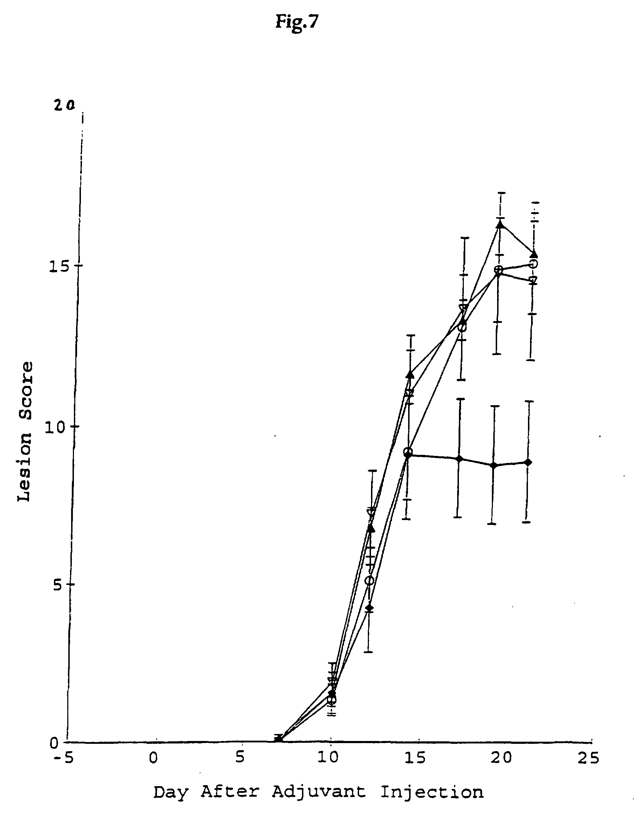

Figure 7 illustrates the effect of treatment, starting on day 10, on lesion score in the rat arthritis model. Open circles represent PBS/Tween 5ml/kg, po, bid. Open triangles represent Cyclosporin A 0.3mg/kg, po, bid. Solid triangles represent compound 1 10mg/kg, po, bid. Solid diamonds represent combined compound 1 at 10mg/kg and Cyclosporin A 0.3mg/kg, administered po, bid.

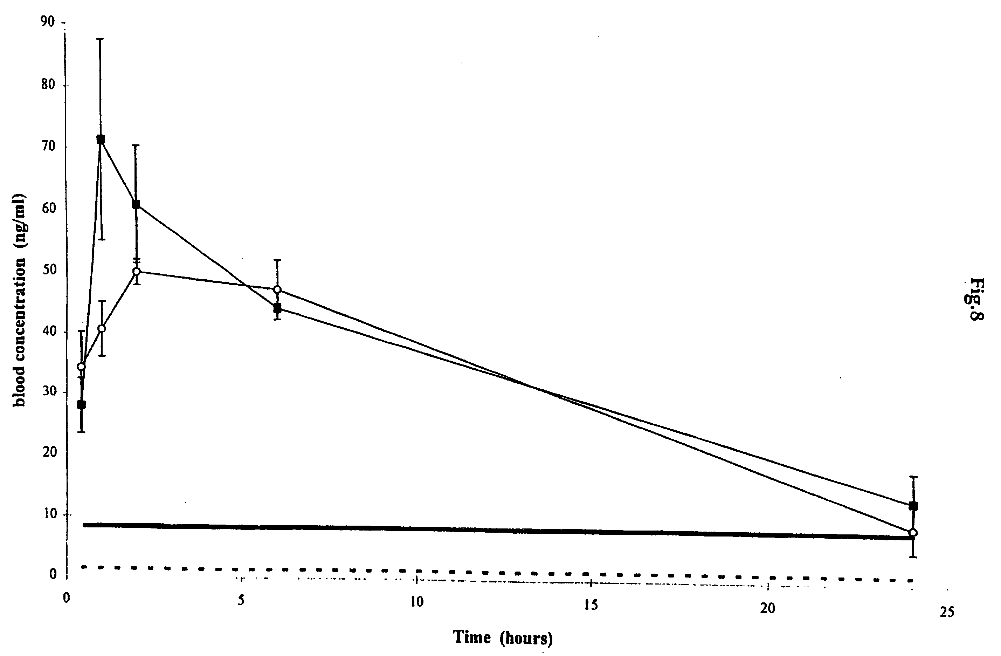

Figure 8 illustrates the blood concentration of Compound 1 against time following oral administration to animals at 10mg/kg as a suspension in PBS/Tween +/- Cyclosporin A (Conc*) (n=6). Thin line represents IC₅₀ of compound 1. Thick line represents 6 x IC₅₀ of compound 1. Open circles represent compound 1 without Cyclosporin A (N=6). Solid squares represent compound 1 with Cyclosporin A (N=6).

### Examples.

### Rat adjuvant-induced arthritis model.

### Materials and Methods.

### Preparation of adjuvant.

100mg of heat-killed *Mycobacterium butyricum* (DIFCO) was placed in a 30ml glass bottle and 20ml of light white mineral oil (Sigma) was added. A 5mg/ml suspension of adjuvant was made by sonication using a probe (Sonics & Materials) at a power output of approximately 50 watts for 15 minutes with stirring.

### Induction of arthritis.

A subcutaneous administration of 0.1ml adjuvant suspension was made at the base of the tail of each male Lewis rat (weighing 180-200g) under light halothane anaesthesia, using a 25G x 5/8" needle and 1ml glass Hamilton syringe.

### Pathometrics.

The onset and progression of arthritis was monitored by measuring bodyweight, right and left hind paw volumes and scoring for any lesions in the tail, ears and paws. Lesions occurring at the tail base injection site were discounted. A number of measurements of normal bodyweights and paw volumes were done before onset of clinical arthritis to establish baseline values.

The same Sartorius balance (model LC4800P) was used throughout the study. Mean of 10 readings was recorded. Paw volume was measured by immersing the paw, up to the hair line, in a beaker of water and weighing the displaced volume. The volume of displaced water was assumed to be equal to the paw volume as 1ml of water weighs approximately 1g.

The severity of secondary lesions was assessed subjectively using the following criteria: Paws-
0 = no lesions
1 = 1 or 2 small local red spots
2 = more than 2 red spots; larger spots; small increase in paw size
3 = more intense erythema; moderate increase in paw size
4 = more intense erythema; severe increase in paw size
5 = more intense erythema; large paw size; broken skin Ears and tails-
0 = no lesions
1 = small erythema; 1 or 2
2 = large erythema; 2 or more; tail appears nodular or deformed.

Mean bodyweight, mean paw volume and mean lesion score for each treatment group were calculated and the data for test compounds were compared to those for vehicle control. The significance of any difference was tested by analysis of variance and, if appropriate, Dunnett's t-test.

### Test compounds

### Cyclosporin used was Cyclosporin A (Sandimmun™; Sandoz)

Compound 1 (3R-(2,2-Dimethyl-1S-methylcarbamoyl-propylcarbamoyl)-5-methyl-2S-2-propenyl-hexanohydroxamic acid) is decribed in Example 5 of WO-A-9421625. The reader is referred thereto for the method of preparation.
Compound 2 ([4-(N-Hydroxyamino)-2R-isobutyl-3S-(thiophenylthiomethyl) succinyl]-L-phenylalanine-N-methylamide) is described in Example 2 of WO-A-9005719. The reader is referred thereto for the method of preparation.

### Treatment groups.

On day 10, animals with the biggest change in mean paw volumes (as compared with day 1 values) were eliminated and the rest were randomised in treatment groups of 9. This was designed to distribute the most and least severely affected animals equally amongst the different groups. Treatments were started on day 10 and were repeated as scheduled. Drugs were formulated fresh for each administration.

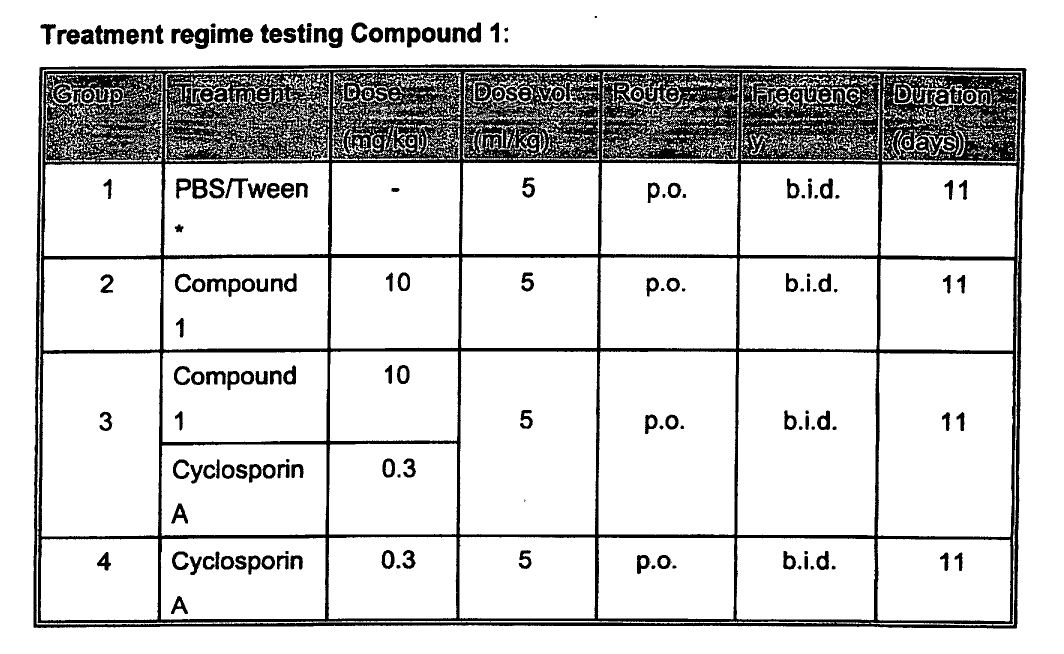

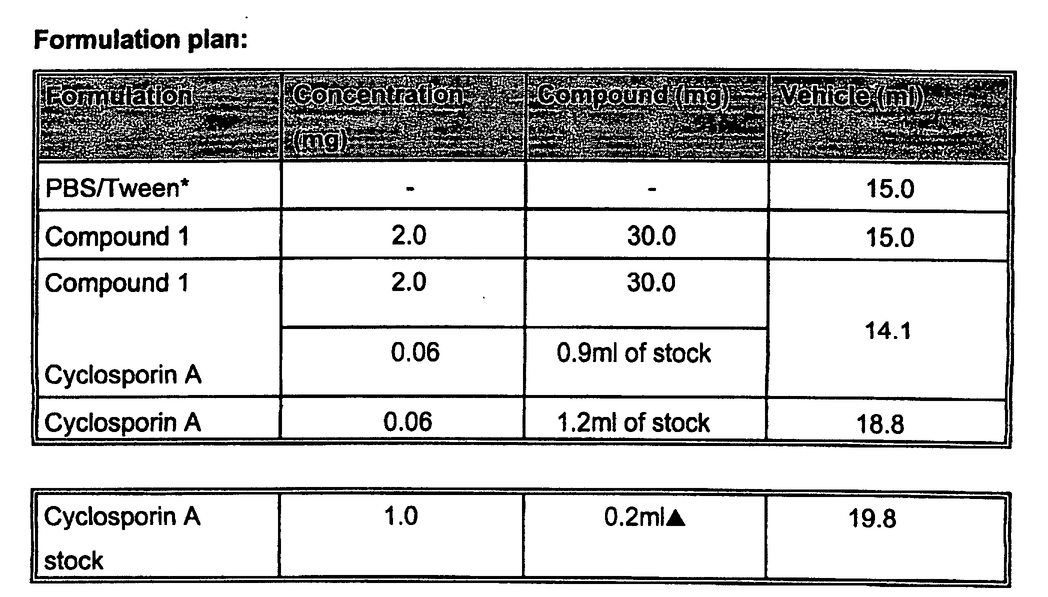

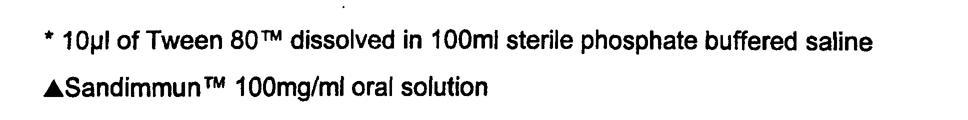

Compound 1 was dispersed by sonication in 14.1ml of vehicle and then made up to volume with 0.9ml of cyclosporin stock emulsion; Concentration value refers to the combined volume.

### Ex vivo bioassay

Compound 1(10mg/kg)±CyA (0.3mg/kg) [in PBS/0.01% Tween 80™] was administered to healthy male Lewis rats (as above). Blood samples (0.5ml into a syringe containing 0.1 ml acid citrate dextroset as anticoagulant) were removed from the ventro-lateral tail vein of the halothane anaestetised rat at t= prebleed, 0.5, 1, 2, 6 and 24 hrs. 3mls methanol was added to each blood sample, mixed and centrifuged at 3000 r.p.m. for 15 minutes (using Beckman GPKR centrifuge). 2.0ml of the methanol supematant was removed and evaporated dry. The pellet was redisolved in 200µl DMSO (dimethylsulphoxide). An aliquot of the solubilised pellet was assayed against fibroblast collagenase using ¹⁴C collagen as substrate, as follows:
- 15µl: fibroblast collagenase at suitable dilution.
- 100µl: collagenase assay buffer*
- 10µl: solubilised pellet
- 75µl: ddH₂O
- 100µl: ¹⁴C collagen
† Acid citrate dextrose, pH4.5 (13.65g citric acid, 25g tri-sodium citrate & 20g d-glucose per litre of water)
* (50mM Tris (pH 7.4), 5mM CaCl₂, 0.05% Brij, 0.02% NaN₃)

Samples were incubated overnight at 37°C and undigested substrate removed by centrifugation at 11,600 r.p.m. for 15 mins (using Heraeus Biofuge 15). 200µl of supernatant was tested by liquid scintillation spectrophotometry.

A calibration curve for the test compound of interest was run alongside the blood samples. The concentration of the test compound in the blood was then calculated from the calibration curve.

### Example 1.

### Dose ranging study of CyA to establish the ineffective dose.

A dose ranging study, testing the effects of cyclosporin A at 0.1, 0.3, 1, 5 and 10 mg/kg, po, bid, in the rat arthritis model described above, was set up. Figures 1, 2 and 3 illustrate the effect of treatment, starting on day 10 post adjuvant administration, on mean paw volume. Figure 1 represents 5mg/kg dosage; Figure 2 represents 1mg/kg dosage and; Figure 3 represents the 0.3mg/kg dosage. The 10mg/kg dosage results were similar to those of the 5mg/kg dosage results and, the 0.1mg/kg dosage results were similar to the 0.3mg/kg dosage results (i.e. ineffective).

In the above rat adjuvant-induced arthritis model, Cyclosporin A was found to be ineffective at reducing the paw volume (swelling) at doses at or below 0.3mg/kg when administered po, bid.

Compound 1 was found to be ineffective at reducing the paw volume (swelling) at doses at or below 10mg/kg when administered po, bid. (data not shown)

### Example 2.

### Combined CyA (0.3mg/kg) and Compound 1 (10mg/kg) po, bid, administration test study.

Cyclosporin A was administered at the ineffective dose of 0.3mg/kg, po, bid.
Compound 1 was administered at the ineffective dose of 10mg/kg, po, bid.

The control vehicle (PBS/Tween) was administered at 5ml/kg, po, bid.
The "combined" test comprised CyA (0.3mg/kg, po, bid) & Compound 1 (10mg/kg, po, bid).

Data is expressed as Group Mean ± SEM using Last Observation Carried Forward (LOCF) analysis to maintain "n" of 9 for all data points. Where there was evidence of activity, data for test compounds were compared to those for vehicle control using "oneway anova" analysis of variance and (if appropriate) Dunnett's t-test.

The vehicle control, Compound 1(10mg/ml), and Cyclosporin A (0.3mg/ml) showed very little or no effect on arthritis.

The combined Compound 1 (10mg/ml) and cyclosporin A (0.3mg/ml) formulations inhibited the vehicle control increase in mean paw volume by 56% (p≤0.5)(see Figures 4 and 5). The beneficial effects of these treatments were also evident in the attenuation of bodyweight loss and lesion score (see Figures 6 and 7)

### Example 3.

### Bioassay study to establish the effects of the oily solution vehicle of CyA on the bioavailability of Compound 1.

Cyclosporin A is formulated in an oil-based vehicle solution. This experiment was designed to identify whether or not CyA and Compound 1 act synergistically in alleviating the symptoms of arthritis, or whether the vehicle in which CyA is formulated, serves to enhance the bioavailability of Compound 1.

Figure 8 illustrates that the blood concentration of Compound 1 is equivalent whether or not Compound 1 is administered with CyA.

### Example 4.

### Combined CyA (0.3mg/kg) and Compound 2(5mg/kg) po, bid, administration

A different matrix metalloproteinase inhibitor compound, Compound 2, when administered at 5mg/kg ip, bid, was also found to act in synergy with cyclosporin A at 0.3mg/kg ip, bid.

## Claims

1. The use of a cyclosporin and a matrix metalloproteinase inhibitor in the preparation of an agent for treating mammals suffering from diseases or conditions mediated by excessive metalloproteinase activity, or for use in transplantation therapy.

2. The use of a cyclosporin and a matrix metalloproteinase inhibitor in the preparation of an agent for treating arthritis by separate, simultaneous or sequential administration of said matrix metalloproteinase inhibitor and said cyclosporin.

3. The use as claimed in claim 2, wherein the matrix metalloproteinase inhibitor and/or the cyclosporin are at doses which are respectively ineffective to alleviate arthritis when administered alone but are effective to do so when co-administered.

4. A pharmaceutical product comprising a matrix metalloproteinase inhibitor and a cyclosporin for separate, simultaneous or sequential use in treating arthritis.

5. A pharmaceutical product as claimed in claim 4, wherein the individual doses of said matrix metalloproteinase inhibitor and said cyclosporin present in the product are respectively ineffective to alleviate arthritis when administered alone for the treatment of arthritis but are effective to do so when co-administered.

6. A use or pharmaceutical product as claimed in any of claims 1 to 5, wherein the cyclosporin is selected from the group consisting of: cyclosporin A, cyclosporin G, rapamycin and FK506.

7. A use or pharmaceutical product as claimed in any of claims 1 to 5, wherein the matrix metalloproteinase inhibitor is selected from the group consisting of: 3R-(2,2-Dimethyl-1S-methylcarbamoyl-propylcarbamoyl)-5-methyl-2S-2-propenyl-hexanohydroxamic acid and [4-(N-Hydroxyamino)-2R-isobutyl-3S-(thiophenylthiomethyl) succinyl]-L-phenylalanine-N-methylamide.

8. A use or pharmaceutical product as claimed in any of claims 1 to 5, wherein the matrix metalloproteinase inhibitor is: N²-[3S-Hydroxy-4-(N-hydroxyamino)-2R-isobutylsuccinyl]-L-tert-leucine-N¹-methylamide.

## Patentansprüche

1. Verwendung eines Cyclosporins und eines
Matrixmetalloproteinase-Inhibitors zur Herstellung eines Mittels zur Behandlung von Säugetieren, die unter Krankheiten oder Umständen leiden, die durch übermäßige Metalloproteinase-Aktivität vermittelt werden, oder zur Verwendung in der Transplantationstherapie.

2. Verwendung eines Cyclosporins und eines
Matrixmetalloproteinase-Inhibitors zur Herstellung eines Mittels zur Behandlung von Arthritis durch einzelne, gleichzeitige oder sequenzielle Verabreichung des Matrixmetalloproteinase-Inhibitors und des Cyclosporins.

3. Verwendung gemäß Anspruch 2, wobei der
Matrixmetalloproteinase-Inhibitor und/oder das Cyclosporin in Dosen vorliegen, die jeweils nicht wirksam sind, Arthritis zu lindern, wenn sie allein verabreicht werden, aber wirksam sind, wenn sie zusammen verabreicht werden.

4. Pharmazeutisches Produkt, das einen
Matrixmetalloproteinase-Inhibitor und ein Cyclosporin für die einzelne, gleichzeitige oder sequenzielle Verwendung zur Behandlung von Arthritis umfasst.

5. Pharmazeutisches Produkt gemäß Anspruch 4, wobei die individuellen Dosen des Matrixmetalloproteinase-Inhibitors und des Cyclosporins, die in dem Produkt vorhanden sind, jeweils unwirksam sind, Arthritis zu lindern, wenn sie allein zur Behandlung von Arthritis verabreicht werden, aber wirksam sind, wenn sie gleichzeitig verabreicht werden.

6. Verwendung oder pharmazeutisches Produkt gemäß einem der Ansprüche 1 bis 5, wobei das Cyclosporin aus der Gruppe ausgewählt wird, die aus Cyclosporin A, Cyclosporin G, Rapamycin und FK506 besteht.

7. Verwendung oder pharmazeutisches Produkt gemäß einem der Ansprüche 1 bis 5, wobei der Matrixmetalloproteinase-Inhibitor aus der Gruppe ausgewählt wird, die aus 3R-(2,2-Dimethyl-1S-methylcarbamoyl-propylcarbamoyl)-5-methyl-2S-2-propenyl-hexanohydroxamsäure und [4-(N-Hydroxyamino)-2R-isobutyl-3S-(thiophenylthiomethyl)succinyl]-L-phenylalanin-N-methylamid besteht.

8. Verwendung oder pharmazeutisches Produkt gemäß einem der Ansprüche 1 bis 5, wobei der Matrixmetalloproteinase-Inhibitor N²-[3S-Hydroxy-4-(N-hydroxyamino]-2R-isobutylsuccinyl]-L-tert-leucin-N¹-methylamid ist.

## Revendications

1. Utilisation d'une cyclosporine et d'un inhibiteur de métalloprotéinase de matrice dans la préparation d'un agent pour traiter des mammiferes souffrant de maladies ou de pathologies à médiation par une activité de métalloprotéinase excessive, ou à utiliser dans une thérapie de transplantation.

2. Utilisation d'une cyclosporine et d'un inhibiteur de métalloprotéinase de matrice dans la préparation d'un agent pour traiter l'arthrite par administration séparée, simultanée ou successive dudit inhibiteur de métalloprotéinase de matrice et de ladite cyclosporine.

3. Utilisation selon la revendication 2, dans laquelle l'inhibiteur de métalloprotéinase de matrice et/ou la cyclosporine sont à des doses qui sont respectivement inefficaces pour soulager l'arthrite quand ils sont administrés seuls mais qui sont efficaces pour ce faire lorsqu'ils sont administrés conjointement.

4. Produit pharmaceutique comprenant un inhibiteur de métalloprotéinase de matrice et une cyclosporine pour une utilisation séparée, simultanée ou successive dans le traitement de l'arthrite.

5. Produit pharmaceutique selon la revendication 4, dans lequel les doses individuelles dudit inhibiteur de métalloprotéinase de matrice et de ladite cyclosporine présents dans le produit sont respectivement inefficaces pour soulager l'arthrite quand elles sont administrées seules pour le traitement de l'arthrite mais sont efficaces pour ce faire lorsqu'elles sont administrées conjointement.

6. Utilisation ou produit pharmaceutique selon l'une quelconque des revendications 1 à 5, dans lequel la cyclosporine est choisie dans le groupe constitué par la cyclosporine A, la cyclosporine G, la rapamycine et FK506.

7. Utilisation ou produit pharmaceutique selon l'une quelconque des revendications 1 à 5, dans lequel l'inhibiteur de métalloprotéinase de matrice est choisi dans le groupe constitué par l'acide 3R-(2,2-diméthyl-1S-méthylcarbamoylpropylcarbamoyl)-5-méthyl-2S-2-propénylhexanohydroxamique et le [4-(N-hydroxyamino)-2R-isobutyl-3S-(thiophénylthiométhyl)succinyl]-L-phénylalanine-N-méthylamide.

8. Utilisation ou produit selon l'une quelconque des revendications 1 à 5, dans lequel l'inhibiteur de métalloprotéinase de matrice est le N²-[3S-hydroxy-4-(N-hydroxyamino)-2R-isobutylsuccinyl]-L-tert-leucine-N¹-méthylamide.
